# EUROPÄISCHE PATENTSCHRIFT

(11) **EP 1 180 093 B1**
(45) Veröffentlichungstag und Bekanntmachung des Hinweises auf die Patenterteilung: **01.10.2003**
(21) Anmeldenummer: 00917077.0
(22) Anmeldetag: 12.04.2000
(51) Int. Cl.: C07C 211/51

(54) **PHOSPHORSÄURESALZ EINES AROMATISCHEN DIAMINS**
PHOSPHORIC ACID SALT OF AN AROMATIC DIAMINE
SEL D'ACIDE PHOSPHORIQUE D'UNE DIAMINE AROMATIQUE

(30) Priorität: 17.04.1999 DE 19917526
(43) Veröffentlichungstag der Anmeldung: 20.02.2002
(73) Patentinhaber: Boehringer Ingelheim Pharma GmbH & Co.KG, 55218 Ingelheim am Rhein (DE)
(72) Erfinder: SCHNEIDER, Heinrich, (DE)
(74) Vertreter: Laudien, Dieter, Dr.
(86) Internationale Anmeldenummer: EP0003248
(87) Internationale Veröffentlichungsnummer: WO00063156

(56) Entgegenhaltungen:
- EP-A- 0 465 121
- GB-A- 2 122 607

## Beschreibung

Die vorliegende Erfindung betrif#t ein neues Salz von N-Methyl-o-phenylendiamin.

### Hintergrund der Erfindung

Aromatische Amine stellen wichtige Zwischenverbindungen ftir die Synthese von Farbstoffen, Antioxidantien und insbesondere Pharmazeutika dar. Besonders aromatische 1,2-Diamine sind für die Herstellung von aromatischen Heterocyclen, insbesondere solchen mit zwei Hetero-Stickstofrätomen, wie Benzünidazole von großer technischen Bedeutung. Gerade letztere und deren Salze sind wegen ihrer phannakologischen Vielfältigkeit von hohem Interesse.

Es ist jedoch bekannt, daß aromatische Amine, wie z.B. Anilin, stark oxidationsempfindlich sind. Dies erschwert insbesondere die Lagerung dieser Verbindungen. Besonders augenscheinlich tritt diese Eigenschaft bei flüssigen oder in einer Flüssigkeit gelösten aromatischen Aminen auf. Denn bereits in Gegenwart geringer Spuren an Sauerstoff oder Licht färben sich diese Lösungen binnen kurzem tief schwarz. Diese hohe OxidationsempfindKchkeit der aromatischen Amine führt dazu, daß sie weiteren chemische Reaktionen nur frisch gereinigt, in der Regel frisch destilliert, zugeführt werden können. Die Oxidationsempfindlichkeit dieser Substanzen kann reduziert werden, indem die aromatischen Amine in ihre Salze überführt werden. Jedoch sind auch die Salze in der Regel immer noch so oxidationsempfindlich, daß sie z.B. unter Sauerstoüausschluß gelagert werden müssen. Auch neigen derartige Salze zur Hygroskopie. Die gebräuchlichsten Salze sind die Hydrohalogenide, insbesondere die Hydrochloride.

Von besonderer Oxidationsempfindlichkeit sind die Phenylendiamine. So beschreibt beispielsweise bereits O. Fischer in den Berichten der deutschen Chemischen Gesellschaft, Band 25 (III), Seite 2841-2842, daß sich das ölige N-Methyl-o-phenylendiamin "außerordentlich schnell dunkel färbt und nach ganz kurzer Zeit selbst in dunklen, wohl verschlossenen Flaschen vollständig schwarz wird." Selbst das Dihydrochlorid stellt nach O. Fischer eine "gegen Luft und Licht ziemlich empfindliche" Substanz dar (O. Fischer, den Berichten der deutschen Chemischen Gesellschaft, Band 25, Seite 2842). Ähnliches gilt für andere aus dem Stand der Technik bekannte Salze des N-Methyl-o-phenylendiamins, für Salze des o-Phenylendiamins selbst und für andere Salze von mono- di- und tri-N-alkylierten Phenylendiaminen.

Neben der hohen Oxidations- und Lichtempfindlichkeit und mitunter ausgeprägten Hygroskopie der Hydrohalogenide der besagten Phenylendiamine besitzen diese Verbindungen zur Verwendung in großtechnischen Verfahren weitere bedeutende Nachteile. Da die Salze bei der weiteren Umsetzung in der Regel *in* situ in die freie Base überführt werden müssen, entsteht als Nebenprodukt die entsprechende Halogenwasserstoffsäure. Dies ist besonders dann von Nachteil, wenn der Reaktionsansatz eine Temperatur aufweist, bei der die Halogenwasserstoffsäure gasförmig anfällt. Da diese Säuren korrosionsfördernd, stark ätzend und in Folge toxisch sind, müssen sie sorgfältig aus dem Reaktionsgeschehen abgeführt werden. Dies ist mitunter nur unter einem beträchtlichen technischen Aufwand möglich, der von hohen Kosten begleitet wird.

Aber nicht nur die Verwendung, sondern auch die Herstellung der Salze der Phenylendiamine mit Halogenwasserstoffsäuren ist großtechnisch mit einigen Schwierigkeiten behaftet. Am zweckmäßigsten sollte der Halogenwasserstoff gasförmig dem entsprechenden Phenylendiamin zugeführt werden, um Ausbeuteverluste zu minimieren. Dies ist jedoch verfahrenstechnisch nur sehr aufwendig möglich, so daß die Salze aus den wässrigen Halogenwasserstoffsäuren gebildet werden und damit hohe Ausbeuteverluste in Kauf genommen werden müssen.

### Beschreibung der Erfindung

Es ist eine Aufgabe der vorliegenden Erfindung, die aus dem Stand der Technik bei der Herstellung, Lagerung und Verwendung N-Methyl-o-phenylendiamin bekannten Schwierigkeiten zu minimieren.

Es ist eine weitere Aufgabe der Erfindung N-Methyl-o-phenylendiamin in eine gut handhabbare, wenig oxidations- und/oder licht-empfindliche und/oder nicht hygroskopische, lagerfähige Form zu überführen, die derart ist, daß die freien Basen für Folgereaktionen schnell zugänglich sind.

### Detaillierte Beschreibung der Erfindung

Überraschenderweise wurde gefunden, daß für das Salz von N-Methyl-O-phenylendiamin und Phosphorsäure die aus dem Stand der Technik für andere derartige Salze bekannten Nachteile und damit verbundenen Schwierigkeiten nicht oder nur in deutlichem geringerem Ausmaß zutrifft.

Damit löst die vorliegende Erfindung die ihr gestellte Aufgabe dadurch, daß sie N-Methyl-o-phenylendiamin in ein Phosphorsäuresalz gemäß Formel I überführt. 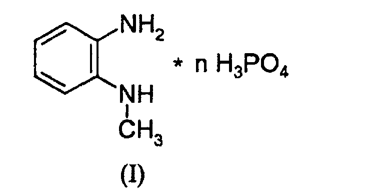 worin n eine Zahl zwischen 0.5 und 1.0 ist.

Unter dem Begriff "Phosphorsäuresalz" werden alle Salze des N-Methyl-o-phenylendiamins mit ortho-Phosphorsäure (H₃PO₄) verstanden. Das Verhältnis von einem Molekül H₃PO₄ zu einem Molekül N-Methyl-o-phenylendiamin gemäß Formel I wird im statistischen Mittel durch den Faktor n beschrieben.

Bevorzugt ist ein Salz der oben beschriebenen Art mit n von 0.7 bis 0.8, insbesondere bevorzugt ist n gleich 0.75 bis 0.78.

In einer besonders bevorzugten Ausführungsformen ist das erfindungsgemäße Phosphorsäuresalz des N-Methyl-o-phenylendiamins wasserfrei oder weist nur geringe Spuren von Wasser auf. Der Wassergehalt des Salzes liegt in der Regel zwischen 1,0 und 1,5 Gew.%.

Die Vorteile des erfindungsgemäßen Phosphorsäuresalzes des N-Methyl-o-phenylendiamins bestehen zum einen in seiner Oxidationsbeständigkeit, Lichtunempfindlichkeit und seinem nicht hygroskopischen Verhalten. Dadurch kann es ohne größeren Aufwand gut gelagert werden und ohne weitere Maßnahmen - mit der optionalen Ausnahme der Überführung in die freie Base - in weiteren Reaktionen eingesetzt werden. Zum anderen ist seine Herstellung großtechnisch überraschend einfach, da die verwendete Phosphorsäure kristallin eingesetzt werden kann. Auch entstehen bei der weiteren Verwendung dieses Phosphorsäuresalzes selbst bei erhöhten Temperaturen keine ätzenden und die Korrosion fördernden Gase, unter Reaktionsbedingungen.

Eine weiterer Aspekt der vorliegenden Erfindung betrifft die großtechnische Herstellung des oben beschriebenen N-Methyl-o-phenylendiamin-Phosphorsäuresalzes.

Das Salz ist einfach und in hohen Ausbeuten zugänglich, indem N-Methyl-o-phenylendiamin in einem mit Wasser oder Alkohol mischbaren Lösungsmittel, wie z.B. C₁ bis C₋₅₋Alkohol, bevorzugt Ethanol, Propanol oder Isopropanol gelöst wird. Diese Mischung wird mit kristalliner Phosphorsäure oder einer Phosphorsäurelösung in Mengen von 75 ― 100 Mol% zur Reaktion gebracht. Die Phosphorsäure kann dabei in Wasser, einem C₁- bis C-₅-Alkohol, wie Methanol, Ethanol, Propanol, Isopropanol oder Gemischen daraus gelöst bzw. suspendiert werden. Bevorzugt werden als Lösungsmittel Ethanol, Propanol oder Isopropanol eingesetzt.

Das N-Methyl-o-phenylendiamin-Phosphorsäure-Salz fällt entweder aus dem Lösungsmittel aus oder kann ggf. durch Zugabe unpolarer Lösungsmittel wie z. B. von aliphatischen oder aromatischen Kohlenwasserstoffen oder Ethem ausgefällt werden.

In einem bevorzugten Verfahren wird wasserfreie Phosphorsäure eingesetzt.

Eine alternative Ausführungsform der Erfindung betrifft Verfahren, in denen N-Methyl-o-phenylendiamin *in situ* durch Reduktion des o-Nitro-N-methylanilins hergestellt wird.

Die Reduktion des o-Nitro-N-methylanilins kann gemäß aus dem Stand der Technik bekannten Verfahren durchgeführt werden. Bevorzugtes Reduktionsmittel ist Wasserstoff unter Verwendung eines Hydrier-Katalysators in einem Alkohol als Lösungsmittel, bevorzugt Ethanol, Propanol oder Isopropanol. Nach Abtrennung des Katalysators kann die so erhaltene Lösung mit Phosphorsäure oder einer Phosphorsäurelösung zur Reaktion gebracht werden. Bevorzugt wird dabei eine Suspension von kristalliner, wasserfreier Phosphorsäure in dem zur Hydrierung verwendeten Alkohol eingesetzt.

### Beispiel 1

In einem 1200 1-VA-Hydrierer wird ein Gemisch aus 190 kg o-Nitro-N-methylanilin, 0,95 kg Palladium-Kohle-Katalysator (10 % Pd) und 570 I Ethanol bei 60 - max. 85°C und einem Wasserstoffdruck von 2 - 6 barÜ bis zum Stillstand der Wasserstoffaufnahme hydriert.
In einen weiteren 1200 1-Apparat werden 99 kg kristalline Phosphorsäure und 238 1 Ethanol vorgelegt und dazu unter Rühren der Inhalt des Hydrierapparats unter Abfiltrieren des Katalysators einfließen gelassen.

Man wäscht Filter und abfiltrierten Katalysator mit 95 1 Ethanol nach, kühlt auf 10 ― 20°C ab und rührt noch 30 ― 60 min nach. Das Produkt wird abzentrifugiert, mit 238 1 Ethanol gewaschen und bei 40 ― 60°C im Vakuum getrocknet.
Ausbeute : 230 kg N-Methylphenylendiamin, 0.77 Phosphat (92 % d. Th.)
Das Produkt enthält typischerweise 1.0 ― 1.5 % Wasser.
Laut Titration berechnet sich ein Gehalt von 61,0 % N-Methylphenylendiamin und von 37,7 % Phosphorsäure.
Elementaranalyse: C: 42,00 % H: 6.34 % N: 14,00 % P: 11,92 %

### Beispiel 2

In einem 1200 1-VA-Hydrierer wird ein Gemisch aus 190 kg o-Nitro-N-methylanilin, 0,95 kg Palladium-Kohle-Katalysator (10 % Pd) und 5701 Isopropanol bei 60 ― max. 85°C und einem Wasserstoffdruck von 2 ― 6 barÜ bis zum Stillstand der Wasserstoffaufnahme hydriert.

In einen weiteren 1200 1-Apparat werden 99 kg kristalline Phosphorsäure und 238 1 Isopropanol vorgelegt und dazu unter Rühren der Inhalt des Hydrierapparats unter Abfiltrieren des Katalysators einfließen gelassen.

Man wäscht Filter und abfiltrierten Katalysator mit 95 1 Isopropanol nach, kühlt auf 10 ― 20°C ab und rührt noch 30 ― 60 min nach. Das Produkt wird abzentrifugiert, mit 238 1 Isopropanol gewaschen und bei 40 ― 60°C im Vakuum getrocknet.
Ausbeute : 225 kg N-Methylphenylendiamin, 0.77 Phosphat (90 % d. Th.)
Laut Titration berechnet sich ein Gehalt von 61,1 % N-Methylphenylendiamin und von 37,6 % Phosphorsäure.
Elementaranalyse: C: 41,98 % H: 6.35 % N: 14,04 % P: 11,93 %

## Patentansprüche

1. Phosphorsäuresalz von N-Methyl-o-phenylendiamin.

2. Phosphorsäuresalz von N-Methyl-o-phenylendiamin nach Anspruch 1, **gekennzeichnet durch** die allgemeine Formel I 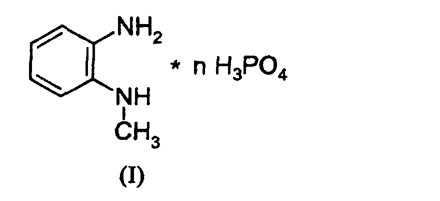 worin n eine Zahl zwischen 0.5 und 1.0 ist.

3. Phosphorsäuresalz von N-Methyl-o-phenylendiamin gemäß Anspruch 2, **dadurch gekennzeichnet, daß** n zwischen 0.7 und 0.8 liegt.

4. Phosphorsäuresalz von N-Methyl-o-phenylendiamin gemäß einem der Ansprüche 2 oder 3, **dadurch gekennzeichnet, daß** n zwischen 0.75 und 0.78 liegt.

5. Verfahren zur Herstellung eines Phosphorsäuresalzes von N-Methyl-o-phenylendiamin gemäß einem der vorangegangenen Ansprüche 1 bis 4, **dadurch gekennzeichnet, daß** N-Methyl-o-phenylendiamin nach einem der vorangegangenen Ansprüche in einem Lösungsmittel gelöst ist und mit kristalliner oder gelöster Phosphorsäure versetzt wird.

6. Verfahren nach Anspruch 5 zur Herstellung eines Phosphorsäuresalzes von N-Methyl-o-phenylendiamin gemäß einem der vorangegangenen Ansprüche 1 bis 4, **dadurch gekennzeichnet, daß** N-Methyl-o-phenylendiamin *in situ* hergestellt wird.

7. Verfahren nach Anspruch 6 zur Herstellung eines Phosphorsäuresalzes von N-Methyl-o-phenylendiamin gemäß einem der vorangegangenen Ansprüche 1 bis 4, **dadurch gekennzeichnet, daß** das o-N-Methylphenylendiamin *in situ* aus o- Nitro-N-methylanilin hergestellt wird.

8. Verfahren nach einem der Ansprüche 5 bis 7 zur Herstellung eines Phosphorsäuresalzes von N-Methyl-o-phenylendiamin gemäß einem der vorangegangenen Ansprüche 1 bis 4, **dadurch gekennzeichnet, daß** das Lösungsmittel ein C₁- bis C₅-Alkohol ist.

9. Verfahren nach Anspruch 8 zur Herstellung eines Phosphorsäuresalzes von N-Methyl-o-phenylendiamin gemäß einem der vorangegangenen Ansprüche 1 bis 4, **dadurch gekennzeichnet, daß** das Lösungsmittel für N-Methyl-o-phenylendiamin und für die Phosphorsäure Ethanol, Propanol oder Isopropanol ist.

10. Verfahren nach den Anspruch 9 zur eines Phosphorsäuresalzes von N-Methyl-o-phenylendiamin gemäß einem der vorangegangenen Ansprüche 1 bis 4, **dadurch gekennzeichnet, daß** das Lösungsmittel für N-Methyl-o-phenylendiamin und für die Phosphorsäure Ethanol ist.

## Claims

1. Phosphoric acid salt of N-methyl-o-phenylenediamine.

2. Phosphoric acid salt of N-methyl-o-phenylenediamine according to claim1, **characterised by** general formula I 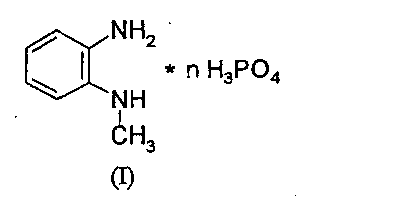 wherein n is a number between 0.5 and 1.0.

3. Phosphoric acid salt of N-methyl-o-phenylenediamine according to claim 2, **characterised in that** n is between 0.7 and 0.8.

4. Phosphoric acid salt ofN-methyl-o-phenylenediamine according to one of claims 2 and 3, **characterised in that** n is between 0.75 and 0.78.

5. Process for preparing a phosphoric acid salt of N-methyl-o-phenylenediamine according to one of the preceding claims 1 to 4, **characterised in that** N-methyl-o-phenylenediamine according to one of the preceding claims is dissolved in a solvent and mixed with crystalline or dissolved phosphoric acid.

6. Process according to claim 5 for preparing a phosphoric acid salt of N-methyl -o-phenylenediamine according to one of the preceding claims 1 to 4, **characterised in that** N-methyl-o-phenylenediamine is prepared *in situ.*

7. Process according to claim 6 for preparing a phosphoric acid salt of N-methyl -o-phenylenediamine according to one of the preceding claims 1 to 4, **characterised in that** the O-N-methylphenylenediamine is prepared *in situ* from o-nitro-N-methylaniline.

8. Process according to one of claims 5 to 7 for preparing a phosphoric acid salt of N-methyl-o-phenylenediamine according to one of the preceding claims 1 to 4, **characterised in that** the solvent is a C₁- to C₅-alcohol.

9. Process according to claim 8 for preparing a phosphoric acid salt of N-methyl-o-phenylenediamine according to one of the preceding claims 1 to 4, **characterised in that** the solvent for N-methyl-o-phenylenediamine and for the phosphoric acid is ethanol, propanol or isopropanol.

10. Process according to claim 9 for preparing a phosphoric acid salt of N -methyl-o-phenylenediamine according to one of the preceding claims 1 to 4, **characterised in that** the solvent for N-methyl-o-phenylenediamine and for the phosphoric acid is ethanol.

## Revendications

1. Sel d'acide phosphorique de la N-méthyl-o-phénylènediamine.

2. Sel d'acide phosphorique de la N-méthyl-o-phénylènediamine selon la revendication 1, **caractérisé par** la formule généraleI 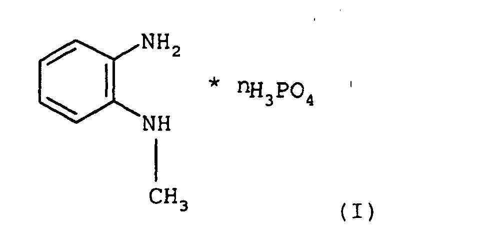 où n est un nombre compris entre 0,5 et 1,0.

3. Sel d'acide phosphorique de la N-méthyl-o-phénylènediamine selon la revendication 2, **caractérisé en ce que** n est compris entre 0,7 et 0,8.

4. Sel d'acide phosphorique de la N-méthyl-o-phénylènediamine selon l'une quelconque des revendications 2 ou 3, **caractérisé en ce que** n est compris entre 0,75 et 0,78.

5. Procédé de préparation d'un sel d'acide phosphorique de la N-méthyl-o-phénylènediamine selon l'une quelconque des revendications précédentes 1 à 4, **caractérisé en ce que** la N-méthyl-o-phénylènediamine selon l'une quelconque des revendications précédentes est dissoute dans un solvant et est mélangée avec de l'acide phosphorique cristallin ou dissous.

6. Procédé, selon la revendication 5, de préparation d'un sel d'acide phosphorique de la N-méthyl-o-phénylènediamine selon l'une quelconque des revendications précédentes 1 à 4, **caractérisé en ce que** la N-méthyl-o-phénylènediamine est préparée *in situ*.

7. Procédé, selon la revendication 6, de préparation d'un sel d'acide phosphorique de la N-méthyl-o-phénylènediamine selon l'une quelconque des revendications précédentes 1 à 4, **caractérisé en ce que** la N-méthyl-o-phénylènediamine est préparée *in situ* à partir de la O-nitro-N-méthylaniline.

8. Procédé, selon l'une quelconque des revendications 5 à 7, de préparation d'un sel d'acide phosphorique de la N-méthyl-o-phénylènediamine selon l'une quelconque des revendications précédentes 1 à 4, **caractérisé en ce que** le solvant est un alcool en C₁ à C₅.

9. Procédé, selon la revendication 8, de préparation d'un sel d'acide phosphorique de la N-méthyl-o-phénylènediamine selon l'une quelconque des revendications précédentes 1 à 4, **caractérisé en ce que** le solvant pour la N-méthyl-o-phénylènediamine et pour l'acide phosphorique est l'éthanol, le propanol ou l'isopropanol.

10. Procédé, selon la revendication 9, de préparation d'un sel d'acide phosphorique de la N-méthyl-o-phénylènediamine selon l'une quelconque des revendications précédentes 1 à 4, **caractérisé en ce que** le solvant pour la N-méthyl-o-phénylènediamine et pour l'acide phosphorique est l'éthanol.
